# EUROPEAN PATENT APPLICATION

(11) **EP 3 915 993 A1**
(43) Date of publication of application: **01.12.2021**
(21) Application number: 20745058.6
(22) Date of filing: 22.01.2020
(51) Int. Cl.: C07D 487/04, A61K 31/4985, A61P 1/16, A61P 31/12, A61P 31/16, A61P 31/18, A61P 31/20, A61P 31/22

(54) **CRYSTAL FORM OF 1,2,3-TRIAZOLO[1,5-A]PYRAZINES DERIVATIVE AND PREPARATION METHOD FOR CRYSTAL FORM**

(30) Priority: 25.01.2019 CN 201910072048
(71) Applicant: Jiangsu Hengrui Medicine Co., Ltd., Lianyungang, Jiangsu 222047 (CN); Shanghai Hengrui Pharmaceutical Co., Ltd, Shanghai 200245 (CN)
(72) Inventor: HAN, Long, Shanghai 200245 (CN); SHAO, Qiyun, Shanghai 200245 (CN); FENG, Jun, Shanghai 200245 (CN); HE, Feng, Shanghai 200245 (CN); MA, Yahui, Shanghai 200245 (CN); ZHAO, Miaomiao, Lianyungang, Jiangsu 222047 (CN); DU, Zhenxing, Lianyungang, Jiangsu 222047 (CN); WANG, Jie, Lianyungang, Jiangsu 222047 (CN)
(74) Representative: dompatent von Kreisler Selting Werner - Partnerschaft von Patent- und Rechtsanwälten mbB
(86) International application number: PCT/CN2020/073802
(87) International publication number: WO 2020/151746

(57) **Abstract**

The present invention provides a crystal form of a 1,2,3-triazolo[1,5-*a*]pyrazines derivative and a preparation method for the crystal form. Specifically, the present invention provides a crystal form of a compound (*S*)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dimethylformamide and a preparation method for the crystal form. The prepared crystal form has good stability and clinical application value.

## Description

The present application claims the priority of Chinese Patent Application CN201910072048.6 filed on January 25, 2019, which is incorporated herein by its entirety.

### Technical Field

The present disclosure provides a crystal form of a compound (S)-*N⁵-*(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide and a preparation method for the crystal form.

### Background

PCT/CN2018/097170 (filed on 26 July, 2018) described a compound (S)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide, and the pharmacodynamic experiment showed that the compound had obvious inhibitory effect on the normal assembly of HBV capsid protein, with good pharmacokinetic absorption and high bioavailability. At the same time, the new compound had no or little effect on the proliferation inhibition of HepG2 cells in vitro, showing good safety.

The polymorph is a phenomenon that there are two or more different spatial arrangements of solid materials, which have different physical and chemical properties. The bioavailability of drugs in the same class may be different due to the different arrangement of different crystal forms. Meanwhile, in view of the importance of the crystal form of the solid drug and its stability in clinical treatment, it is of great significance to study the polymorph of the compound (S)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide to obtain the crystal form with high purity and stable chemical properties for the development of drugs suitable for industrial production and with good biological activity.

### Summary of the invention

The present disclosure provides a crystal form A of a compound (S)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide, wherein the X-ray powder diffraction pattern represented by a diffraction angle 2*θ* has characteristic peaks at 13.197, 14.239, 15.839, 17.680, 19.080, 19.780 and 22.539.

In an alternative embodiment, said crystal form A is characterized in that the X-ray powder diffraction pattern represented by a diffraction angle 2*θ* has characteristic peaks at 13.197, 14.239, 15.320, 15.839, 17.680, 19.080, 19.780, 22.539, and 25.519.

In some embodiments, said crystal form A is characterized in that the X-ray powder diffraction pattern represented by a diffraction angle 2*θ* has characteristic peaks at 11.022, 13.197, 14.239, 15.320, 15.839, 17.680, 19.080, 19.780, 20.879, 22.539, 25.519, and 26.041.

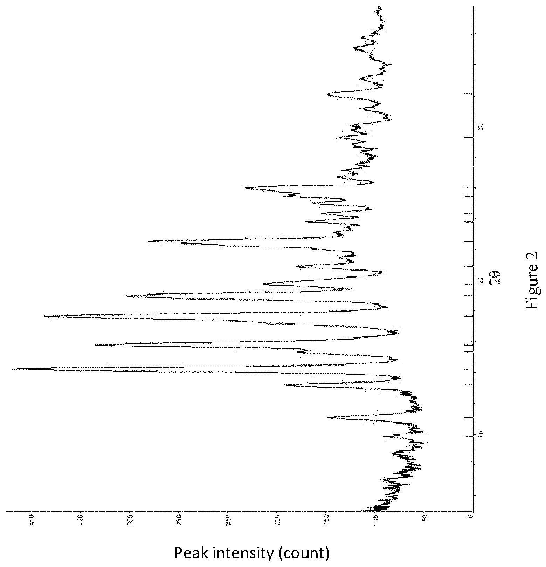

In some other embodiments, said crystal form A is characterized in that the X-ray powder diffraction pattern represented by a diffraction angle 2*θ* has been shown in Figure 2.

The present disclosure further provides a method for preparing the crystal form A of the compound (S)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide, including:
(a) adding the compound (S)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide into the solvent (I) and being dissolved by stirring or heating, wherein said solvent (I) is selected from at least one of ethyl acetate, dichloromethane, isopropanol and isopropyl ether, preferably isopropanol/ isopropyl ether, ethyl acetate/ n-hexane or dichloromethane/ isopropyl ether.
(b) precipitating the crystal by stirring.

In some embodiments, said solvent (I) used in this method is selected from a mixed solvent of isopropanol/ isopropyl ether. A volume ratio of isopropanol to isopropyl ether is 2:1 - 1:10, which can be 2:1, 1:1, 1:2, 1:3, 1:4, 1:5, 1:6, 1:7, 1:8, 1:9, 1:10 and any ratio between any two values, preferably 1:3, 1:4 or 1:5.

In some embodiments, said solvent (I) used in this method is selected from a mixed solvent of ethyl acetate/ n-hexane. A volume ratio of ethyl acetate to n-hexane is 2:1 - 1:10, which can be 2:1, 1:1, 1:2, 1:3, 1:4, 1:5, 1:6, 1:7, 1:8, 1:9, 1:10 and any ratio between any two values, preferably 1:3, 1:4 or 1:5.

In some other embodiments, said solvent (I) used in this method is selected from a mixed solvent of dichloromethane/ isopropyl ether. A volume ratio of dichloromethane to isopropyl ether is 1:5 - 1:30, which can be 21:5, 1:6, 1:7, 1:8, 1:9, 1:10, 1:11, 1:12, 1:13, 1:14, 1:15, 1:16, 1:17, 1:18, 1:19, 1:20, 1:22, 1:23, 1:24, 1:25, 1:26, 1:27, 1:28, 1:29, 1:30 and any ratio between any two values, preferably 1:20, 1:22 or 1:25.

In some embodiments, the volume (ml) of said solvent (**I**) used in this method is 1-20 times of the weight (g) of the compound, which can be 1, 2, 3, 4, 5, 6, 7, 8, 9, 10, 11, 12, 13, 14, 15, 16, 17, 18, 19 or 20 times and any value between any two values.

In another aspect, the present disclosure further provides a crystal form B of the compound (S)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide, wherein the X-ray powder diffraction pattern represented by a diffraction angle 2*θ* has characteristic peaks at 6.273, 12.680, 14.178, 15.475, 17.685, 19.045 and 22.450.

In an alternative embodiment, said crystal form B is characterized in that the X-ray powder diffraction pattern represented by a diffraction angle 2*θ* has characteristic peaks at 6.273, 11.687, 12.680, 14.178, 15.475, 17.198, 17.685, 19.045 and 22.450.

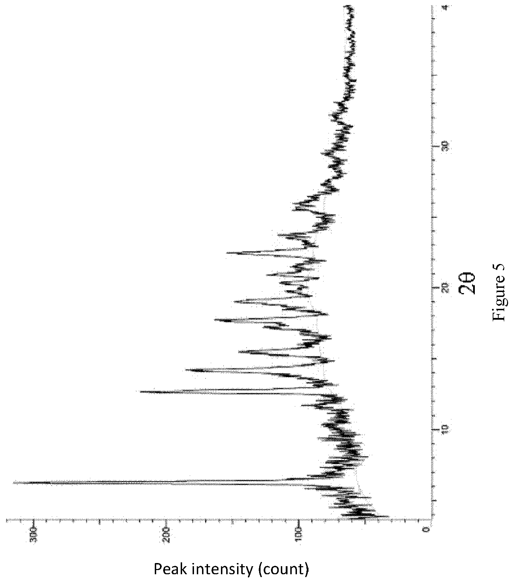

In other embodiments, said crystal form B is characterized in that the X-ray powder diffraction pattern represented by a diffraction angle 2*θ* has been shown in Figure 5.

The present disclosure further provides a crystal form C of the compound (S)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide, wherein the X-ray powder diffraction pattern represented by a diffraction angle 2*θ* has characteristic peaks at 6.326, 10.317, 11.833, 12.826, 13.805, 20.529 and 23.773.

In an alternative embodiment, said crystal form C is characterized in that the X-ray powder diffraction pattern represented by a diffraction angle 2*θ* has characteristic peaks at 6.326, 10.317, 11.833, 12.826, 13.805, 15.499, 16.875, 18.546, 20.529 and 23.773.

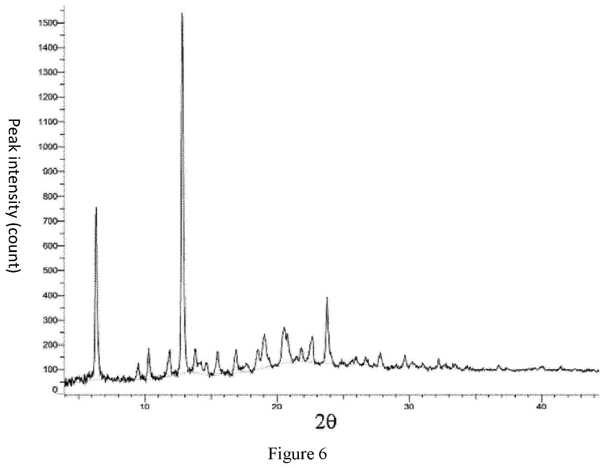

In some other embodiment, said crystal form C is characterized in that the X-ray powder diffraction pattern represented by a diffraction angle 2*θ* has been shown in Figure 6.

The present disclosure also provides a crystal form D of the compound (S)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide, wherein the X-ray powder diffraction pattern represented by a diffraction angle 2*θ* has characteristic peaks at 10.296, 12.415, 15.542, 18.521, 19.298, 23.004 and 25.956.

In an alternative embodiment, said crystal form D is characterized in that the X-ray powder diffraction pattern represented by a diffraction angle 2*θ* has characteristic peaks at 10.296, 12.415, 15.542, 16.660, 18.521, 19.298, 20.058, 23.004 and 25.956.

Preferably, said crystal form D is characterized in that the X-ray powder diffraction pattern represented by a diffraction angle 2*θ* has characteristic peaks at 10.296, 12.415, 15.542, 16.660, 18.521, 19.298, 20.058, 21.214, 22.039, 23.004 and 25.956.

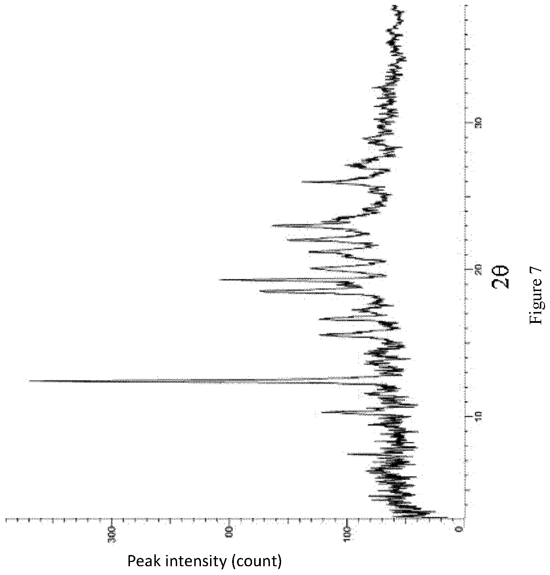

In some other embodiment, said crystal form D is characterized in that the X-ray powder diffraction pattern represented by a diffraction angle 2*θ* has been shown in Figure 7.

The present disclosure further provides a pharmaceutical composition prepared from any one of the above crystal forms. Furthermore, the pharmaceutical composition also contains a pharmaceutically acceptable carrier, diluent or excipient.

The present disclosure further provides a pharmaceutical composition containing the crystal form of the above compound and a pharmaceutically acceptable carrier, diluent or excipient.

The present disclosure further provides the use of the above crystal form of the compound, or the pharmaceutical composition in the preparation of drugs for the prevention and/ or treatment of viral infectious diseases, wherein said virus can be one or more of hepatitis B virus, influenza virus, herpes virus and AIDS virus, and the disease can be one or more of hepatitis B, influenza, herpes and AIDS.

The present disclosure further provides the use of the compound of the above crystal form or the pharmaceutical composition in the preparation of drugs for capsid protein inhibitors.

The present disclosure further provides a method for preventing and/ or treating viral infectious diseases, which includes administering a therapeutically effective dose of a compound of the above crystal form to a patient in need thereof, wherein said virus can be one or more of hepatitis B virus, influenza virus, herpes virus and AIDS virus, and the disease can be one or more of hepatitis B, influenza, herpes and AIDS.

According to the definition of hygroscopicity characteristics and hygroscopic weight gain in "9103 guiding principles for hygroscopicity of drugs" in the fourth volume of Chinese Pharmacopoeia (2015 Edition),
Deliquescence: sufficient moisture is absorbed to form a liquid;
Extremely hygroscopic: hygroscopic weight gain is no less than 15%;
Hygroscopic: hygroscopic weight gain is less than 15% but no less than 2%;
Slightly hygroscopic: hygroscopic weight gain is less than 2% but no less than 0.2%;
No or little hygroscopicity: hygroscopic weight gain is less than 0.2%.

The crystal form A of said (S)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide according to the present disclosure has a hygroscopic weight gain of 0.1% under the condition of 70% RH, which is less than 0.2%, indicating no or little hygroscopicity.

Furthermore, the crystal form A according to the present disclosure has a hygroscopic weight gain of 0.21% under the condition of 90% RH, indicating slight hygroscopicity, or no/ little hygroscopicity.

"The X-ray powder diffraction pattern" described in the present disclosure is obtained by using Cu-Kα radiation measurement.

The preparation method of the crystal form described in the present disclosure also includes the steps of filtering, washing or drying, or the like.

The "X-ray powder diffraction pattern or XRPD" described in the present disclosure is measured based on Bragg formula 2d sin *θ* = nλ (where, λ is the wavelength of the X-ray, λ=1.54060Å, the diffraction order n is any positive integer, generally taking the first diffraction peak, n = 1) that is satisfied when the X-ray is incident on an atomic plane of a crystal or part of the crystal sample with d lattice plane spacing at grazing angle θ (a residual angle of the incident angle, also known as a Bragg angle).

"2*θ* or 2*θ* degree" described in the present disclosure refers to a diffraction angle, where *θ* is the Bragg angle, in ° or degree; the angle 2*θ* for each characteristic peak has an error range of ±0.20, which can be -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, - 0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20.

The "crystal surface spacing or crystal surface spacing (d value)" described in the present disclosure refers to parallel hexahedron unit in the space lattice divided by three unit vectors a, b, and c which are unparallel and connect adjacent two lattice points. The space lattice is divided into a set of straight-line grid according to the determined parallelepiped unit, which is called space lattice or lattice. The lattice and crystal lattice reflect the periodicity of crystal structure with geometric points and lines respectively, and the plane spacing (that is, a distance between two adjacent parallel planes) of different lattice planes is different; the unit is Å or angstrom.

The "differential scanning calorimetry or DSC" described in the present disclosure refers to the measurement of the temperature difference and heat-flux difference between the sample and the reference during the heating process or thermostatic process of the sample, so as to characterize all physical and chemical changes related to the thermal effect, and obtain the phase change information of the sample.

The drying temperature in the present disclosure is generally 20°C to 100°C, preferably 25°C to 70°C, which drying can be carried out under normal pressure or reduced pressure (vacuum drying). Preferably, the drying is carried out under reduced pressure.

The chemical reagents and biological reagents used in the present disclosure can be commercially available. Compound B in the present disclosure is carried out according to the method in PCT/ CN2018 / 097170 (filed on July 26, 2018), whose contents related to pharmacological efficacy and animal in vivo research are introduced into the present disclosure for illustration.

The reaction process in the Examples is monitored by thin layer chromatography (TLC), and the developing agent used in the reaction, the eluent system used in column chromatography for purifying the compound and the developing agent system for thin layer chromatography include: A: dichloromethane/ methanol system, B: n-hexane/ ethyl acetate system, C: petroleum ether/ ethyl acetate system, wherein the volume ratio of the solvent can be adjusted according to the polarity of the compounds, or by the addition of a small amount of basic or acidic reagent such as triethylamine and acetic acid. Test conditions for the instruments used for experiments in the present disclosure are as follows.

XRPD indicates X-ray powder diffraction detection: the determination is carried out by BRUKER D8 X-ray diffractometer, and the specific information is collected: Cu anode (40 kV, 40 mA), Cu-Kα1 ray (λ=1.54060Å), Kα2 ray (λ=1.54439Å), Kβ ray (λ=1.39222Å), scanning range (2q range): 3-64°, a scanning step length of 0.02 and a slit width (collimator) of 1.0 mm. A step-by-step scanning method is carried out with a number of scanning steps of 3, scanning range per step of 19°, starting degree of 5°, termination degree 48, and time duration per step of 75 s.

DSC indicates differential scanning calorimetry: the determination is carried out with METTLER TOLEDO DSC 3+ differential scanning calorimeter, with a heating rate of 10°C/ min, a specific temperature range which refers to the corresponding map (25-300 or 25-350°C), and a nitrogen purging speed of 50 ml/ min.

TGA indicates thermogravimetry: the determination is carried out by METTLER TOLEDO TGA type 2 thermal gravimetric analyzer, with the heating rate of 10°C/ min, the specific temperature range which refers to the corresponding map (25-300°C), and the nitrogen purge speed of 20 ml/ min.

DVS indicates dynamic vapor sorption: the determination is carried out by SMS DVS Advantage at 25°C with a humidity change of 50%-95%-0%-95%-50%, and the step of 10% (the last step is 5%) (The specific range of humidity is subject to the corresponding spectrum, and the methods listed here are those used in most cases), and the judgment standard is that dm/ dt is not more than 0.02%.

HPLC is performed by Agilent 1200 DAD high pressure liquid chromatographic instrument (Sunfire C18, column: 150 × 6 mm) and Waters 2695-2996 high pressure liquid chromatographic instrument (gimini C18, column: 150 × 6 mm).

The structure of the compound is determined by nuclear magnetic resonance (NMR) or/ and mass spectrometry (MS). The NMR shift (δ) is given in units of 10⁻⁶ (ppm). The determination by NMR is carried out with Bruker AVANCE-400 nuclear magnetic instrument using DMSO-d₆, CDCl₃ and CD₃OD as the solvent, and the internal standard is TMS; the determination by MS is carried out with Finnigan lcqad (ESI) mass spectrometer (manufacturer: Thermo, model: Finnigan LCQ advantage MAX).

### Brief description of the drawings

Figure 1: amorphous XRPD pattern of a compound.
Figure 2: XRPD spectrum of a crystal form A of the compound.
Figure 3: XRPD pattern of the crystal form A in Example 5.
Figure. 4: X-ray powder diffraction pattern of the crystal form A of the compound before and after DVS.
Figure 5: XRPD pattern of a crystal form B of the compound.
Figure 6: XRPD pattern of a crystal form C of the compound.
Figure 7: XRPD pattern of a crystal form D of the compound.

### Detailed description of the preferred embodiment

The present disclosure will be explained in detail in combination with the Examples or experimental Examples. The Examples or experimental Examples in the present disclosure are merely used to illustrate the technical solutions in the present disclosure, but not to limit the essence and scope of the present disclosure.

### Example 1: Preparation of (S)-N⁵-(3,4-difluorophenyl)-6-methyl-N³-((R)-1,1,1-trifluoropropan-2-yl)-6,7-dihydro-[1,2,3]triazolo[1,5-a]pyrazine-3,5(4H)-dicarbonamide (compound B).

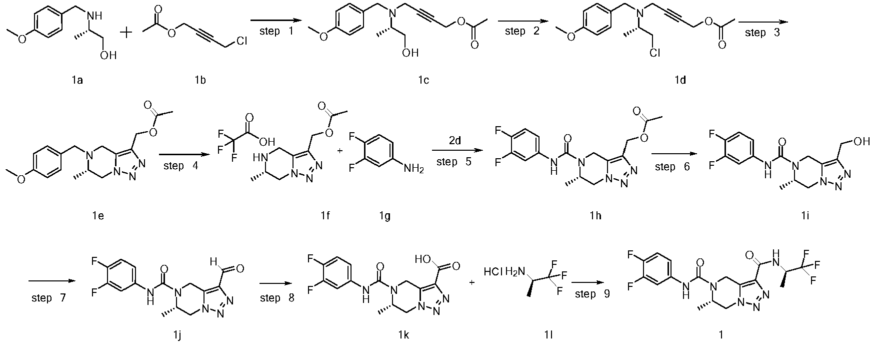

### Step 1

### (S)-4-((1-hydroxypropyl-2-yl)(4-methoxybenzyl)amino))butyl-2-yne-1-yl acetate 1c

Compound **1a** (3.00 g, 15.00 mmol, prepared by the well-known method *"*Bioorganic & Medicinal Chemistry Letters, 2015, 25(5), 1086-1091") was dissolved in 60 ml of dioxane, and then 4-chlorobutyl-2-yne-1-yl acetate **1b** (5.73 g, 39.00 mmol, prepared by the well-known method"Journalof Medicine Chemistry, 2014, 57(9),3687-3706") was added followed by triethylamine (4.7 g, 46.00 mmol), and the reaction was stirred at 60°C for 12 hours. The reaction solution was filtered to obtain the filtrate which was then concentrated under reduced pressure, and the resulting residue was purified by silica gel column chromatography with the eluent system A, obtaining compound **1c** (2.20 g, yield: 42.2%).
MS m/z (ESI): 306.2 [M+1].

### Step 2

### (S)-4-((1-chloropropyl-2-yl)(4-methoxybenzyl)amino)butyl-2-yne-1-yl acetate 1d

Compound **1c** (2.20 g, 7.20 mmol) and pyridine (854 mg, 10.08 mmol) were dissolved in 30 ml dichloromethane, to which was added slowly sulfoxide chloride (1.50 g, 12.60 mmol) under ice bath, slowly raising to room temperature and stirring for 2 hours. The reaction solution was added with 100 mL of dichloromethane, washed with water (50 mL × 2), dried over anhydrous sodium sulfate and filtrated, and the filtrate was concentrated under reduced pressure to obtain the title compound **1d** (2.20 g), which was directly used for the next reaction without purification.

### Step 3

### (S)-(5-(4-methoxybenzyl)-6-methyl-4,5,6,7-tetrahydro-[1,2,3]triazolo[1,5-a]pyrazin-3-yl)methyl acetate 1e

The crude compound **1d** (2.20 g, 6.79 mmol) was dissolved in 20 ml of N,N-dimethylformamide, and sodium azide (574 mg, 8.83 mmol) was added, reacting at 80°C for 12 hours. The reaction solution was cooled down to room temperature, added with 50 mL of ethyl acetate, washed with water (20 mL × 2), dried over anhydrous sodium sulfate and filtrated, and the filtrate was concentrated under reduced pressure to obtain the residue which was then purified by silica gel column chromatography with the eluent system A, obtaining compound **1e** (1.30 g, yield: 57.9%).
MS m/z (ESI): 331.1 [M+1].

### Step 4

### (S)-(6-methyl-4,5,6,7-tetrahydro-[1,2,3]triazolo[1,5-a]piperazine-3-yl)methyl acetate trifluoroacetate 1f

Compound **1e** (1.30 g, 2.33 mmol) was dissolved in 5 ml of trifluoroacetic acid, heated to 100°C by microwave and reacted for 5 minutes. The reaction solution was cooled down to room temperature and concentrated under reduced pressure to obtain the crude title compound **1f** (1.28 g), which was directly used for the next reaction without purification.

### Step 5

### (S)-(5-((3,4-difluorophenyl)carbamoyl)-6-methyl-4,5,6,7-tetrahydro-[1,2,3]triazolo[1,5-a]pyrazine-3-yl)methyl acetate 1h

Crude compound **1f** (200 mg, 0.62 mmol), compound **1g** (228 mg, 1.90 mmol) and triethylamine (290 mg, 2.86 mmol) were dissolved in 10 ml tetrahydrofuran, and bis(trichloromethyl)carbonate (87 mg, 0.3 mmol) was added under ice water bath, reacting under stirring for 3 hours. The reaction solution was concentrated under reduced pressure, and the resulting residue was purified by silica gel column chromatography with the eluent system C, obtaining compound **1h** (90 mg, yield: 40.1%).
MS m/z (ESI): 366.1 [M+1].

### Step 6

### (S)-N-(3,4-difluorophenyl)-3-(hydroxymethyl)-6-methyl-6,7-dihydro-[1,2,3]triazolo[1,5-a]pyrazine-5(4H)-formamide 1i

Compound **1h** (442 mg, 1.21 mmol) was dissolved in 6 ml of mixed solvent of methanol and water (V: V= 5: 1), and lithium hydroxide (253.86 mg, 6.05 mmol) was added and stirred for 1.5 h. The resction solution was concentrated under reduce pressure, and the resulting resudie was added with 30 mL of ethyl acetate, washed with water (2 mL×2). The organic phase was concentrated under reduce pressure to obtain the crude compound **1i** (391 mg), which was directly used for the next reaction without purification.
MS m/z (ESI): 324.1 [M+1].

### Step 7

### (S)-N-(3,4-difluorophenyl)-3-formoxyl-6-methyl-6,7-dihydro-[1,2,3]triazolo[1,5-a]pyrazine-5(4H)-formamide 1j

The crude compound **1i** (350 mg, 1.1 mmol) was dissolved in 12 mL of dichloromethane, and pyridinium chlorochromate (583.41 mg, 2.71 mmol) and silica gel (550 mg, 100 mesh) were added and stirred for 2 hours. The resultant was filtered and concentrated under reduced pressure to obtain the resudie which was then purified by thin layer chromatography with the developing agent system A, obtaining compound **1j** (60 mg, yield: 17%).
MS m/z (ESI): 322.1 [M+1].

### Step 8

### (S)-((3,4-difluorophenyl)aminocarbonyl)-6-methyl-4,5,6,7-dihydro-[1,2,3]triazolo[1,5-a]pyrazine-3-formic acid 1k

Compound 1J (60 mg, 0.19 mmol) was dissolved in 5 ml of mixed solvent of acetonitrile and water (V:V = 3:2), and aminosulfonic acid (36.26 mg, 0.37 mmol) was added; the solution of sodium chlorite (33.78 mg, 0.37 mmol) dissolved in 2 ml of water was added to the reaction system, and stirred at room temperature for 3 hours. The resultant was added with 1 mL of saturated sodium sulfite solution, adjusted to pH 2 with 1 N HCl, and extracted with ethyl acetate (10 mL×3). The organic phases were combined, and concentrated under reduced pressure to obtain the crude compound **1k** (30 mg), which was directly used for the next reaction without purification.
MS m/z (ESI): 338.4 [M+1].

### Step 9

### (S)-N⁵-(3,4-difluorophenyl)-6-methyl-N³-((R)-1,1,1-trifluoropropropropyl-2-yl)-6,7-dihydro-[1,2,3]triazolo[1,5-a]pyrazine-3,5(4H)-dicarbonamide 1

The crude compound **1k** (50 mg, 148.2 µmol), *O*-(7-azabenzotriazol-1-yl)-*N,N,N',N*'-tetramethylurea hexafluorophosphate (52.32 mg, 222.4 µmol) and *N,N-*diisopropylethylamine (76.64 mg, 593 µmol) were dissolved in 3 ml of *N,N-*dimethylformamide and reacted for 10 minutes. Compound **1l** (33.25 mg, 222.4 µmol, prepared by the method disclosed in the patent application "CN102875270A") was added and reacted under stirring for 2 hours. The reaction solution was concentrated under reduced pressure, and the resulting residue was purified by high efficiency liquid chromatography (separation conditions: chromatographic column: Gilson gx-281, mobile phase: A-water (10 mmol ammonia acetate), B-acetonitrile, flow rate: 18 ml/ min) to obtain 20 mg of crude product which was treated by chiral preparation (separation conditions: chirally preparative column: Shimadzu chiral preparative column: LC-20AP, CHIRALPAK-AY Lux LC Column 250 ^{∗} 21.2 mm, 5 um; Mobile phase: A-n-hexane: B-ethanol (0.1% DEA) = 85: 15, flow rate: 20 ml/ min). The corresponding fractions were collected and concentrated under reduced pressure to obtain product **1** (5 mg) product.
MS m/z (ESI): 433.1[M+1];

Chiral HPLC analysis: retention time: 8.647 min, chiral purity: 99.8% (chromatographic column: AY Phenomenex Lux Amylose-2 150^{∗}4.6 mm, 5 µm; mobile phase: n-hexane/ ethanol (0.1% DEA) = 85/ 15 (V/ V)).

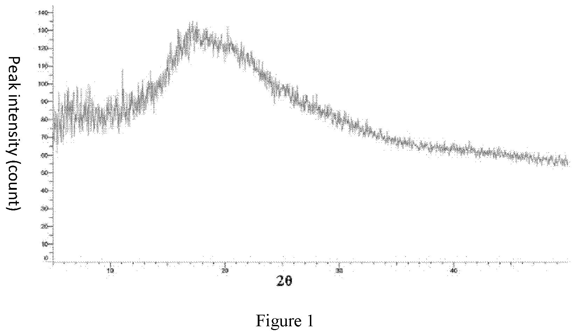

The X-ray powder diffraction showed that this crystal form was amorphous, which XRPD spectrum was shown in Figure 1.
MS m/z (ESI): 439.0 [M+1].
¹H NMR(400MHz, CD₃OD): 7.50-7.48 (m, 1H), 7.18-7.16 (m, 2H), 5.43 (d, 1H), 5.08-5.06 (m, 1H), 4.89-4.87 (m, 1H), 4.74 (d, 1H), 4.60 (d, 1H), 4.49-4.46 (m, 1H), 1.49 (d, 3H), 1.21 (d, 3H).

### Test Example 1: in vitro anti-HBV activity test (quantitative analysis of intracellular HBV DNA)

### 1. Experimental materials and instruments

(1) QIAamp 96 DNA QIAcube HT Kit (Qiagen)
(2) QIAcube HT plasticware (Qiagen)
(3) Hepatitis B Virus Nucleic Acid Quantitative Detection Kit (Tepp biology)
(4) DNA extraction equipment (QIAcube) (Qiagen)
(5) QuantStudio 6 Fiex (ABI, ThermFisher)
(6) Microplate Reader (BMG)
(7) HepG2.2.15 Cells (Shanghai RuiLu Biotechnology Co., Ltd)

### 2. Experimental steps

HepG2.2.15 cells were stable expression cell lines integrated with HBV genome, which can be secreted out of cells through replication, transcription, translation and packaging into virus particles with HBV DNA. In this study, the quantitative PCR method was used to quantitatively analyze the HBV DNA produced by the proliferation of HepG2.2.15 in vitro, thus determining the activity of the compounds in the present disclosure to inhibit HBV DNA replication through inhibiting the assembly of HBV capsid protein.

HepG2.2.15 cells were cultured in DMEM/ high glucose medium (10% FBS, 400 µg/ml G418) and passaged every 3 days. On the day of experiment, fresh cell culture medium was used to prepare cell suspension, which was cultured with 40,000 cells/ well in 96 well plate (Corning, #3599) under 5% carbon dioxide at 37°C. On the second day, the compound was first dissolved in pure DMSO with the concentration of 20 mM, then the first concentration of 2 mM was prepared with DMSO followed by diluted to 8 concentrations by 4 times in turn. The control well was added with 90 µL of DMSO, which was diluted 200 times with DMEM/ high glucose-containing medium. The cell culture plate inoculated on the first day was taken out, and then, the medium in the well plate was sucked out by using the negative pressure suction device. Into each well, the prepared medium containing each concentration of compound was added respectively, which was cultured with 200 µl/ well at 37°C for 72 hours. On the fifth day, the fresh medium containing the same compound was used to exchange the old medium by using the method that was the same as the second day, and then the fresh medium was cultured at 37°C for 72 hours. On the eighth day, the cell culture plate was taken out, centrifuged at 300 g for 3 minutes, and then the supernatant was collected and cultured with 200 ml µL/ well. The extraction of HBV DNA from cell culture supernatant was carried out by using Qiagen automatic DNA extraction equipment, with the method specifically mentioned in reagent and instrument instructions. At last, the extracted DNA was eluted with DNA elution buffer with 100 µL/ well. The extracted DNA was analyzed by HBV DNA quantitative PCR using Hepatitis B virus nucleic acid quantitative detection kit from Tepp biology, with the method specifically mentioned in the instructions of the kit. The quantitative standard curve was determined in parallel experiment using the own standard sample of the kit. Each sample was converted quantitatively based on the standard curve. Finally, the EC50 values of the compounds were calculated by Graphpad Prism software based on the concentrations of the compounds and the corresponding DNA values. Emax is the maximum inhibitory effect value of the compound on HBV DNA replication.

It was determined that compound B in the present disclosure can inhibit in vitro activity of HBV DNA replication through inhibiting the assembly of HBV capsid protein by the above test, and the results were EC₅₀=19 nM, and Emax=100%, indicating an obvious inhibition on HBV DNA replication.

### Test Example 2: effect on proliferation of HepG2 cells in vitro

### 1. Experimental materials and instruments

(1) HepG2 cells (ATCC)
(2) CellTiter-Glo ^{™} Cell proliferation Kit (Promega)
(3) Automatic pipetting workstation (Bravo): Agilent Technologies Corperation
(4) Microplate Reader (VICTOR 3): PerkinElmer Corperation
(5) CO₂ incubator (Fisher Scientific)
(6) Centrifuge (Fisher Scientific)

### 2. Experimental steps

HepG2 cells taken in logarithmic growth stage were prepared into the cell suspension through digestion with trypsin, and then the suspension was cultured with 6,000 cells/ well in 96-well plate (96-well White/Clear Flat Bottom plate) under 5% carbon dioxide at 37°C for 16-20 hours. On the second day, the compound was dissolved in pure DMSO with the concentration of 20 mM, and then the gradient dilution of the compound was carried out by using Automatic pipetting workstation (Bravo) with the dilution times of 3, where there were 8 concentration points for each compound, and the control well was DMSO; next, the compound with each concentration treated by DMSO was diluted 200 times using EMEM (containing 10% FBS) medium. The cell culture plate inoculated on the first day was taken out, and then the medium in the well plate was sucked out by using the negative pressure suction device. Into each well, the prepared medium containing each concentration of compound was added respectively, which was cultured with 100 µl/ well at 37°C for 72 hours. On the fifth day, the 96-well cell plate was taken out, and the freshly prepared CellTiter Glo was added into each well with 100 µL/ well, left to stand for 5-10 minutes, sealed for the bottom of the 96-well plate with a white back cover film (PerkinElmer), placed into the Microplate Reader, and measured for the Luminescence signal by the reader. CC50 values of the compounds were calculated by Graphpad Prism software based on the concentrations of the compounds and the corresponding proliferation inhibition signal values, and the result was CC₅₀> 100 µM, indicating no or little effect on the proliferation inhibition of HepG2 cells in vitro, showing high safety.

### Example 2:

Compound B (200 mg, 0.46 mmol) was added to the mixed solvent of isopropanol and isopropyl ether (V: V= 1: 4), dissolved under heating and stirring, precipitated by stirring, filtered and dried to obtain the product (122 mg, yield: 61%). According to the X-ray powder diffraction test, the XRPD spectrum was shown in Figure 2, which was defined as the crystal form A.

### Example 3:

Compound B (200 mg, 0.46 mmol) was added to 6 ml of the mixed solvent of ethyl acetate and n-hexane (V: V= 1: 4), dissolved under heating and stirring, precipitated by stirring, and filtered, and then the cake was collected and vacuum dried to obtain the product (116 mg, yield: 58%).

The crystal form was determined as the crystal form A by the X-ray powder diffraction.

### Example 4:

Compound B (100 mg, 0.23 mmol) was added to 21 mL of the mixed solution of dichloromethane and isopropyl ether (V: V= 1: 20), dissolved under heating and stirring, slowly cooled down to room temperature, and slowly volatilized to precipitate the solid, and then the cake was collected by filtration and dried to obtain the product (15 mg, yield: 15%).

The crystal form was determined as the crystal form A by XRPD test.

### Example 5:

The crystal form A of compound B (38 g, 87.9 mmol) was added to ether (80 mL), slurried under stirring at room temperature for 16 hours and filtered, and then the cake was washed with ether (30 mL×2) followed by dried to obtain the product (36.3 g, yield: 95.5%).

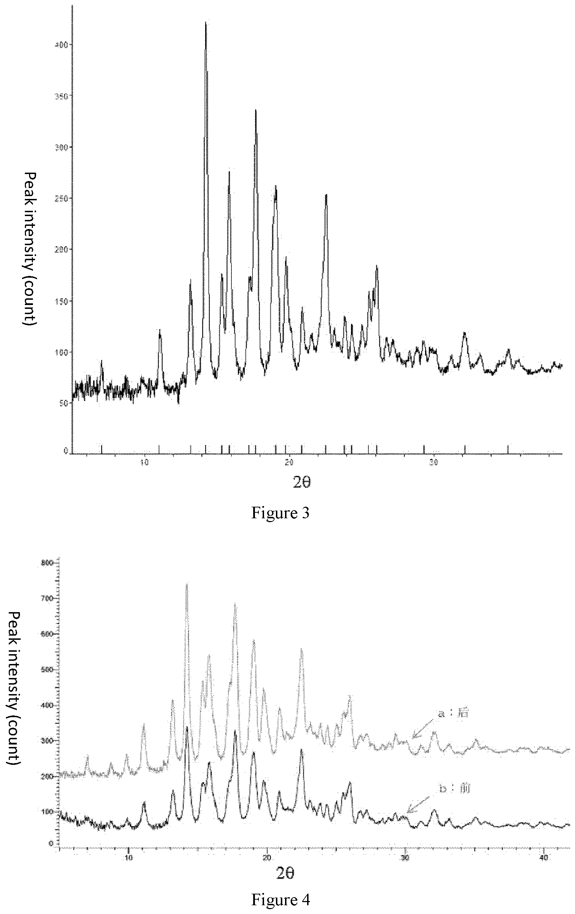

The crystal form was determined as the crystal form A by the X-ray powder diffraction, whose XRPD pattern was shown in Figure 3, in which the positions of characteristic peaks were shown below in Table 1; there were endothermic peaks at 149.39°C and 184.81°C in DSC spectrum; thermogravimetric analysis (TGA) showed that the weight loss was 0.85% between 40°C and 175°C,

**Table 1**

| Peak number | 2*θ* value [° or degree] | D[Å] | relative intensity% |
|---|---|---|---|
| Peak 1 | 7.038 | 12.5488 | 4.40 |
| Peak 2 | 11.022 | 8.0210 | 13.90 |
| Peak 3 | 13.197 | 6.7033 | 28.40 |
| Peak 4 | 14.239 | 6.2149 | 88.30 |
| Peak 5 | 15.320 | 5.7787 | 32.00 |
| Peak 6 | 15.839 | 5.5904 | 88.20 |
| Peak 7 | 17.202 | 5.1506 | 69.40 |
| Peak 8 | 17.680 | 5.0123 | 100.00 |
| Peak 9 | 19.080 | 4.6476 | 64.20 |
| Peak 10 | 19.780 | 4.4847 | 27.20 |
| Peak 11 | 20.879 | 4.2510 | 10.60 |
| Peak 12 | 22.539 | 3.9416 | 56.30 |
| Peak 13 | 23.821 | 3.7323 | 10.20 |
| Peak 14 | 24.340 | 3.6539 | 4.80 |
| Peak 15 | 25.519 | 3.4876 | 51.60 |
| Peak 16 | 26.041 | 3.4190 | 38.40 |
| Peak 17 | 29.338 | 3.0417 | 18.70 |
| Peak 18 | 32.141 | 2.7826 | 11.40 |
| Peak 19 | 35.119 | 2.5532 | 9.10 |

DVS test showed that the sample had the hygroscopic weight gain of about 0.07% under the normal storage condition (i.e. 25°C and 60% RH); the hygroscopic weight gain of about 0.10% under the accelerated test condition (i.e. 70% RH); and the hygroscopic weight gain of about 0.21% under the extreme condition (90% RH). The sample had a desorption process which was consistent with the adsorption process during the humidity change from 0% to 95% RH. The crystal form remained unchanged after DVC detection, as shown in Figure 4 (A was the XRPD pattern after DVS detection, and B was the XRPD pattern before DVS detection).

### Example 6:

The crystal form A of compound B (40 g, 92.5 mmol) was added to 0.5 mL of the mixed solution of ethanol and water (V: V= 2: 3), slurried under stirring at room temperature for 216 hours and filtered, and then, the cake was collected followed by being dried in vacuum to obtain the product (26 g, yield: 65%).

The crystal form was determined as the crystal form A by XRPD test.

### Example 7:

The crystal form A of compound B (40 g, 92.5 mmol) was added to 0.5 mL of cyclohexane, slurried under stirring at room temperature for 216 hours and filtered, and then the cake was collected followed by dried in vacuum to obtain the product (35 g, yield: 87.5%).

The crystal form was determined as the crystal form A by XRPD test.

### Example 8:

The crystal form A of compound B (40 g, 92.5 mmol) was added to 0.6 mL of the mixed solution of acetone and n-heptane (V: V= 1: 5), slurried under stirring at room temperature for 216 hours and filtered, and then the cake was collected followed by dried in vacuum to obtain the product (33 g, yield: 82.5%).

The crystal form was determined as the crystal form A by XRPD test.

### Example 9:

Experimental example 1: investigation of influencing factors

The sample of the crystal form A (Example 5) was spread out, and its stability was investigated under the conditions of heating (40°C, 60°C), illumination (4500 Lux) and high humidity (RH 75%, RH 90%). The sampling was investigated over 30 days.

**Table 2**

| condition | Time (day) | Crystal form A | | | |
|---|---|---|---|---|---|
| | | Color and character | Purity% | Weight gain% | Crystal form |
| start | 0 | white solid | 98.30 | | 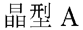 |
| 4500 Lux | 5 | white solid | 98.27 | | NA |
| | 10 | white solid | 98.27 | | NA |
| | 30 | white solid | 98.30 | | No changed |
| 40°C | 5 | white solid | 98.28 | | NA |
| | 10 | white solid | 98.31 | | NA |
| | 30 | white solid | 98.32 | | No changed |
| 60°C | 5 | white solid | 98.26 | | NA |
| | 10 | white solid | 98.27 | | NA |
| | 30 | white solid | 98.30 | | No changed |
| RH 75% | 5 | white solid | 98.26 | 2.28 | NA |
| | 10 | white solid | 98.29 | 2.72 | NA |
| | 30 | white solid | 98.29 | 6.68 | No changed |
| RH 90% | 5 | white solid | 98.26 | 2.93 | NA |
| | 10 | white solid | 98.28 | 3.19 | NA |
| | 30 | white solid | 98.30 | 10.70 | No changed |

| | | | | | |
|---|---|---|---|---|---|
| Note: NA was undetected | | | | | |

The experimental results of the influencing factors in Table 2 showed that the crystal form A had good physical and chemical stability under the conditions of illumination, high temperature of 40°C, high temperature of 60°C, high humidity 75%, and high humidity 90% during storing for 30 days.

### Experimental example 2: long term/ accelerated stability

The crystal form A (Example 5) was placed at 25°C, 60% RH and 40°C, 75% RH to investigate its stability

**Table 3**

| Sample | Conditions | Purity % | | | | |
|---|---|---|---|---|---|---|
| | | start | 1 month | 2 months | 3 months | 6 months |
| Crystal | 25°C, 60%RH | 98.3 | 98.3 | 98.3 | 98.3 | 98.3 |
| form A | 40°C, 75%RH | 98.3 | 98.3 | 98.3 | 98.3 | 98.2 |

The results of long term/ accelerated stability test showed that the crystal form A of compound B had the excellent physical and chemical stability when it was stored for 6 months under the conditions of long-term (25°C, 60% RH) and acceleration (40°C, 75% RH).

### Example 10:

The crystal form A (9.4 mg) of compound B was added into 40 µL of acetonitrile solution and volatilized at room temperature to obtain the product.

The crystal form was determined as the crystal form B by the X-ray powder diffraction, whose XRPD pattern was shown in Figure 5, in which the positions of characteristic peaks were shown below in Table 4.

**Table 4**

| Peak number | 2*θ* value[° or degree] | D[Å] | relative intensity% |
|---|---|---|---|
| peak 1 | 6.273 | 14.07901 | 100.00 |
| peak 2 | 11.687 | 7.56569 | 11.30 |
| peak 3 | 12.680 | 6.97553 | 60.30 |
| peak 4 | 14.178 | 6.2416 | 42.10 |
| peak 5 | 15.475 | 5.72137 | 25.40 |
| peak 6 | 17.198 | 5.15202 | 16.70 |
| peak 7 | 17.685 | 5.01101 | 28.10 |
| peak 8 | 19.045 | 4.65628 | 23.20 |
| peak 9 | 20.328 | 4.36514 | 8.70 |
| peak 10 | 20.938 | 4.23923 | 10.30 |
| peak 11 | 22.450 | 3.95719 | 25.20 |
| peak 12 | 23.695 | 3.75198 | 11.70 |
| peak 13 | 25.986 | 3.42614 | 7.90 |

### Example 11:

The crystal form A (9.4 mg) of compound B was added into 40 µL of nitromethane solution and volatilized at room temperature to obtain the product.

The crystal form was determined as the crystal form C by the X-ray powder diffraction, whose XRPD pattern was shown in Figure 6, in which the positions of characteristic peaks were shown below in Table 5,

**Table 5**

| Peak number | 2*θ* value[° or degree] | D[Å] | relative intensity% |
|---|---|---|---|
| peak 1 | 6.326 | 13.96013 | 45.00 |
| peak 2 | 9.503 | 9.2994 | 3.30 |
| peak 3 | 10.317 | 8.56725 | 7.70 |
| peak 4 | 11.833 | 7.47299 | 6.30 |
| peak 5 | 12.826 | 6.89659 | 100.00 |
| peak 6 | 13.805 | 6.40959 | 6.10 |
| peak 7 | 14.652 | 6.04082 | 3.10 |
| peak 8 | 15.499 | 5.71256 | 5.80 |
| peak 9 | 16.875 | 5.24962 | 5.70 |
| peak 10 | 17.702 | 5.00636 | 2.00 |
| peak 11 | 18.546 | 4.7803 | 5.40 |
| peak 12 | 19.034 | 4.6588 | 8.80 |
| peak 13 | 20.529 | 4.32294 | 9.90 |
| peak 14 | 20.740 | 4.27935 | 6.10 |
| peak 15 | 21.834 | 4.06734 | 3.70 |
| peak 16 | 22.598 | 3.93156 | 6.30 |
| peak 17 | 23.773 | 3.73985 | 18.10 |
| peak 18 | 27.800 | 3.20657 | 3.10 |
| peak 19 | 29.679 | 3.00763 | 3.20 |
| peak 20 | 32.239 | 2.77441 | 2.50 |

### Example 12:

The crystal form A 13.6 mg) of compound B was added into 150 µL of 1,2-dichloroethane solution and volatilized at room temperature to obtain the product.

The crystal form was determined as the crystal form D by the X-ray powder diffraction, whose XRPD pattern was shown in Figure 7, in which the positions of characteristic peaks were shown below in Table 6,

**Table 6**

| Peak number | 2*θ* value[° or degree] | D[Å] | relative intensity% |
|---|---|---|---|
| peak 1 | 10.296 | 8.58513 | 24.20 |
| peak 2 | 12.415 | 7.12415 | 100.00 |
| peak 3 | 15.542 | 5.69685 | 20.70 |
| peak 4 | 16.660 | 5.3169 | 17.70 |
| peak 5 | 17.336 | 5.11109 | 5.60 |
| peak 6 | 18.521 | 4.78683 | 32.40 |
| peak 7 | 19.298 | 4.59579 | 44.20 |
| peak 8 | 20.058 | 4.42325 | 18.00 |
| peak 9 | 20.926 | 4.24179 | 8.20 |
| peak 10 | 21.214 | 4.1847 | 17.70 |
| peak 11 | 22.039 | 4.02997 | 22.00 |
| peak 12 | 23.004 | 3.86309 | 27.40 |
| peak 13 | 25.956 | 3.42999 | 22.10 |
| peak 14 | 27.110 | 3.28655 | 9.50 |

Although the specific embodiments of the present invention have been described above, those skilled in the art should understand that these are only examples, and a variety of changes or modifications can be made to these embodiments without departing from the principle and essence of the present invention. Therefore, the scope of protection of the present invention is defined by the appended claims.

## Claims

1. A crystal form A of a compound (*S*)-*N⁵*-(3,4-difluorophenyl)-6-methyl-*N³*-((*R*)-1,1,1-trifluoropropan-2-yl)-6,7-dihydro-[1,2,3]triazolo[1,5-*a*]pyrazine-3,5(4*H*)-dicarbonamide, wherein the X-ray powder diffraction pattern represented by a diffraction angle 2*θ* has characteristic peaks at 13.197, 14.239, 15.839, 17.680, 19.080, 19.780 and 22.539.

2. The crystal form A according to claim 1, wherein the X-ray powder diffraction pattern represented by a diffraction angle 2*θ* has characteristic peaks at 13.197, 14.239, 15.320, 15.839, 17.680, 19.080, 19.780, 22.539, and 25.519.

3. The crystal form A according to claim 1 or claim 2, wherein the X-ray powder diffraction pattern represented by a diffraction angle 2*θ* has characteristic peaks at 11.022, 13.197, 14.239, 15.320, 15.839, 17.680, 19.080, 19.780, 20.879, 22.539, 25.519, and 26.041.

4. The crystal form A according to any one of claims 1-3, wherein the X-ray powder diffraction pattern represented by a diffraction angle 2*θ* has been shown in Figure 2.

5. A method for preparing the crystal form A according to any one of claims 1-4, comprising:
(a) adding the compound (*S*)-*N⁵*-(3,4-difluorophenyl)-6-methyl-*N³*-((R)-1,1,1-trifluoropropan-2-yl)-6,7-dihydro-[1,2,3]triazolo[1,5-*a*]pyrazine-3,5(4*H*)-dicarbonamide into the solvent (I) and dissolving by stirring or heating, wherein said solvent (I) is selected from at least one of ethyl acetate, dichloromethane, isopropanol and isopropyl ether, preferably isopropanol/ isopropyl ether, ethyl acetate/ n-hexane or dichloromethane/ isopropyl ether,
(b) precipitating the crystal by stirring.

6. The crystal form A according to any one of claims 1-4, wherein it has no or little hygroscopicity at 20.0% RH-80% RH

7. The crystal form A according to any one of claims 1-4, wherein said angle 2*θ* has an error range of ±0.20.

8. A pharmaceutical composition, comprising the crystal form A as defined in any one of claims 1-4, and optionally pharmaceutically acceptable carrier, diluent or excipient.

9. A pharmaceutical composition, which is prepared by the crystal form A as defined in any one of claims 1-4, and optionally pharmaceutically acceptable carrier, diluent or excipient.

10. A use of the crystal form A as defined in any one of claims 1-4, or the composition as defined in claim 8 or 9 in the manufacture of a medicament for the prevention and/ or treatment of viral infectious diseases, wherein said virus is preferably one or more of hepatitis B virus, influenza virus, herpes virus and AIDS virus.
